# EUROPÄISCHE PATENTANMELDUNG

(11) **EP 1 939 195 A1**
(43) Veröffentlichungstag der Anmeldung: **02.07.2008**
(21) Anmeldenummer: 07123142.7
(22) Anmeldetag: 26.07.2001
(51) Int. Cl.: C07D 401/12, A61K 31/47

(54) **Neue Indolderivate und deren Verwendung als Arzneimittel**

(30) Priorität: 28.07.2000 DE 10037310
(62) Teilanmeldung aus: 01969522.0
(71) Anmelder: AEterna Zentaris GmbH, 60314 Frankfurt (DE)
(72) Erfinder: Emig, Peter, 63486, Bruchköbel (DE); Bacher, Gerald, 82110, Germering (DE); Reichert, Dietmar, 63863, Eschau (DE); Baasner, Silke, 61137, Schöneck (DE); Aue, Beate, 63762, Großostheim-Ringheim (DE); Nickel, Bernd, 64367, Mühlthal (DE); Günther, Eckhard, 63477, Maintal (DE)

(57) **Zusammenfassung**

Die Erfindung betrifft neue Indol-Derivate der allgemeinen Formel 1, deren Herstellung und Verwendung als Arzneimittel, insbesondere zur Behandlung von Tumoren.

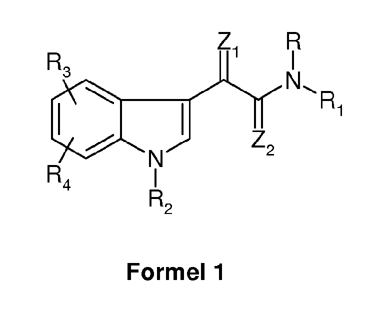

## Beschreibung

Die Erfindung betrifft neue Indol-Derivate der allgemeinen Formel 1, deren Herstellung und Verwendung als Arzneimittel, insbesondere zur Behandlung von Tumoren.

Indol-3-yl-Derivate mit bestimmten 2-, 3-, 4-, und 8-Chinolin-Resten sind in der deutschen Anmeldung DE 198 14 838.0 beschrieben. Indol-3-yl-Derivate mit 5-, 6-oder 7-Chinolin-Substituenten sind in der europäischen Patentanmeldung Nr. 01 969 522.0 beschrieben.

Gemäß einem Aspekt der Erfindung werden neue Indol-Derivate der allgemeinen Formel 1 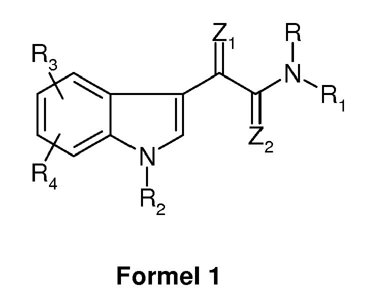 worin
- R: Wasserstoff; geradkettig oder verzweigtes (C₁-C₆)-Alkyl; ein- oder mehrfach durch Aryl substituiertes (C₁-C₆)-Alkyl, wobei der Arylrest unsubstituiert oder ein- bis fünffach gleich oder verschieden mit Halogen, (C₁-C₆)-Alkyl, (C₃-C₇)-Cycloalkyl, Carboxyl, (C₁-C₆)-Alkoxycarbonyl, vorzugsweise tertiärButoxycarbonyl, mit einem oder mehreren Fluoratomen substituiertes geradkettig oder verzweigtes (C₁-C₆)-Alkyl, vorzugsweise Trifluormethyl, Hydroxy, geradkettig oder verzweigtes (C₁-C₆)-Alkoxy, vorzugsweise Methoxy oder Ethoxy; Benzyloxy, oder Aryl-(C₁-C₆)-Alkyl, wobei der Arylrest unsubstituiert oder ein- oder fünffach gleich oder verschieden mit (C₁-C₆)- Alkyl, Halogen oder mit einem oder mehreren Fluoratomen substituiertes geradkettig oder verzweigtes (C₁-C₆)-Alkyl, vorzugsweise Trifluormethyl, substituiert sein kann; geradkettig oder verzweigtes (C₁-C₆)-Alkoxy-(C₁-C₆)-Alkyl, substituiertes oder unsubstituiertes Aryl-(C₁-C₆)-alkyloxycarbonyl, vorzugsweise Benzyloxycarbonyl, geradkettig oder verzweigtes (C₁-C₆)-Alkyloxycarbonyl, geradkettig oder verzweigtes (C₁-C₆)-Alkylcarbonyl, vorzugsweise Acetyl, (C₂-C₆)-Alkenyl, vorzugsweise Allyl, (C₂-C₆)-Alkinyl, vorzugsweise Ethinyl oder Propargyl, oder geradkettiges oder verzweigtes Cyano-(C₁-C₆)-Alkyl, vorzugsweise Cyanomethyl, bedeutet;
- R₁: einen ein oder mehrere Heteroatome ausgewählt aus der Gruppe N, O und S enthaltenden gesättigten, ungesättigten oder aromatischen (C2-C14)-Heterocyclus, welcher mit dem Amidstickstoff direkt oder über eine (C1-C6)-Alkylbrücke verbunden sein kann, wobei der (C₁-C₄)-Alkyl-Rest unsubstituiert oder ein- oder mehrfach gleich oder verschieden mit (C₁-C₆)-Alkyl, Halogen oder Oxo (=O) substituiert sein kann und der 2-, 4-, 5- oder 6-Pyrimidinyl-Rest jeweils unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann, *mit der Maßgabe,* dass R1 nicht für unsubstituiertes 2- oder 4-Pyrimidinyl oder einen ein- oder mehrfach mit der Methylgruppe substituierten 2-Pyrimidinyl-Rest steht; und *ausgenommen* einen 2-, 3-, 4-, 5-, 6-, 7- oder 8-Chinolyl-Rest oder 2-, 3-, 4-, 5-, 6-, 7- oder 8-Chinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, N,N-di-(C₁-C₆)-Alkylamino, Hydroxyl, (C₁-C₆)-Alkoxy, (C6-C14)-Aryl-(C1-C6)-alkyoxy, Carboxy, (C₁-C₆)-Alkoxycarbonyl, (C₁-C₆)-Alkoxycarbonylamino oder ein- oder mehrfach mit Halogen substituiertem (C₁ -C₆)-Alkyl, (C₆-C₁₄)-Aryl, oder (C₆-C₁₄)-Aryl-(C₁-C₆)-alkyl substituiert sein kann; bedeutet;
- R₂: Wasserstoff, (C₁-C₆)-Alkyl, ein- oder mehrfach mit Halogen substituiertes (C₁-C₆)-Alkyl, ein- oder mehrfach mit Phenyl substituiertes (C₁-C₆)-Alkyl, wobei der Phenylrest unsubstituiert oder ein- bis fünffach gleich oder verschieden mit Halogen, vorzugsweise Chlor, Brom oder Jod, (C₁-C₆)-Alkyl, (C₃-C₇)-Cycloalkyl, Carboxyl, (C₁-C₆)-Alkoxycarbonyl, ein- oder mehrfach mit Halogen substituiertem (C₁-C₆)-Alkyl, vorzugsweise Trifluormethyl, Hydroxy, (C₁-C₆)-Alkoxy, vorzugsweise Methoxy oder Ethoxy, Phenyl-(C₁-C₆)-Alkoxy, vorzugsweise Benzyloxy, Nitro, Amino, Mono-(C₁-C₆)-alkylamino, Di-(C₁-C₆)-alkylamino, Mono-(C₃₋₆)-cycloalkylamino, Di-(C₃₋₆)-cycloalkylamino, (C₁-C₆)-Acylamino, Phenyl-(C₁-C₆)-alkylamino, Arylcarbonylamino, Heteroarylcarbonylamino, (C₁-C₆)-Alkylsulfonamido, Arylsulfonamido, Maleinimido, Succinimido, Phthalimido, Benzyloxycarbonylamino (Z-Amino), tert.-Butoxycarbonylamino (BOC-Amino), 9-Fluorenylmethoxy-carbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), mit-NH-CH₂-COOH; -NH-CH(CH₃)-COOH; (CH₃)₂CH-CH₂-CH₂-CH(NH-)-COOH; H₃C-CH₂-CH(CH₃)-CH(NH-)-COOH;
HOH₂C-CH(NH-)-COOH; Phenyl-CH₂-CH(NH-)-COOH; (4-Imidazolyl)-CH₂-CH(NH-)-COOH; HN=C(NH₂)-NH-(CH₂)₃-CH(NH-)-COOH; H₂N-(CH₂)₄-CH(NH-)-COOH;
H₂N-CO-CH₂-CH(NH-)-COOH; HOOC-(CH₂)₂-CH(NH-)-COOH substituiert sein kann;
einen 2-, 3-, 4-, 5-, 6-, 7- und 8-Chinolyl-(C₁-C₆)-Alkyl-Rest, wobei der 2-, 3-, 4-, 5,-, 6- , 7- und 8-Chinolyl-Rest unsubstituiert oder ein- bis sechsfach gleich oder verschieden mit Halogen, (C₁-C₄)-Alkyl oder (C₁-C₄)-Alkoxy substituiert sein kann;
einen 2-, 3- und 4-Pyridyl-(C₁-C₆)-Alkyl-Rest, wobei der 2-, 3- und 4-Pyridyl-Rest unsubstituiert oder ein- bis vierfach gleich oder verschieden mit Halogen, (C₁-C₄)-Alkyl oder (C₁-C₄)-Alkoxy substituiert sein kann;
einen Arylcarbonyl-Rest, wobei der Aryl-Rest unsubstituiert oder ein- bis fünffach gleich oder verschieden mit Halogen, (C₁-C₆)-Alkyl, (C₃-C₇)-Cycloalkyl, Carboxyl, Cyano, (C₁-C₆)-Alkoxycarbonyl, ein- oder mehrfach mit Fluoratomen substituiertes (C₁-C₆)-Alkyl vorzugsweise Trifluormethyl, Hydroxyl, (C₁-C₄)-Alkoxy vorzugsweise Methoxy oder Ethoxy, Aryl-(C₁-C₄)-Alkoxy, vorzugsweise Benzyloxy, substituiert sein kann; bedeutet;
- R₃ und R₄: können an die Indolkohlenstoffatome C-2, C-4, C-5, C-6 oder C-7 gebunden sein und unabhängig voneinder Wasserstoff, Hydroxy, (C₁-C₆)-Alkyl, (C₃-C₇)-Cycloalkyl, (C₁-C₆)-Alkylcarbonyl, (C₁-C₆)-Alkoxy, Halogen, Aryl-(C₁-C₄)-Alkoxy, vorzugsweise Benzyloxy, Nitro, Amino, Mono-(C₁-C₄)-Alkyl-amino, Di-(C₁-C₄)-Alkylamino, (C₁-C₆)-Alkoxycarbonylamino, (C₁-C₆)-Alkoxycarbonylamino-(C₁-C₆)-alkyl, Cyano, geradkettiges oder verzweigtes Cyano-(C₁-C₆)-alkyl, Carboxyl, (C₁-C₄)-Alkoxycarbonyl, mit ein oder mehreren Fluoratomen substituiertes (C₁-C₄)-Alkyl, vorzugsweise Trifluormethylgruppe, Carboxy-(C₁-C₆)-alkyl oder (C₁-C₆)-Alkoxycarbonyl-(C₁-C₆)-alkyl bedeuten;
- Z₁: Sauerstoff oder Schwefel oder geminal verknüpftes Wasserstoff und Hydroxy bedeutet;
- Z₂: Sauerstoff oder Schwefel bedeutet,
deren Tautomere, Stereoisomere, einschließlich der Diastereomere und Enantiomere, sowie die physiologisch verträglichen Salze davon, bereitgestellt.

So lassen sich beispielsweise die erfindungsgemäßen Verbindungen gemäß der allgemeinen Formel (1), welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Des Weiteren können die erfindungsgemäßen Indol-Derivate der allgemeinen Formel (1) in ihre Salze mit anorganischen oder organischen Säuren, insbesondere für die pharmazeutische Anwendung in ihre physiologisch verträglichen Salze, überführt werden. Als Säuren kommen hierfür beispielsweise Salzsäure, Bromwasserstoffsäure, Schwefelsäure, Phosphorsäure, Fumarsäure, Bernsteinsäure, Milchsäure, Äpfelsäure, Embonsäure, Malonsäure, Zitronensäure, Essigsäure, Weinsäure, Trifluoressigsäure, Methansulfonsäure, Sulfoessigsäure oder Maleinsäure in Betracht.

Außerdem lassen sich die erfindungsgemäßen Verbindungen gemäß der Formel (1), falls diese eine ausreichend saure Gruppe wie eine Carboxygruppe enthalten, gewünschtenfalls in ihre Salze mit anorganischen oder organischen Basen, insbesondere für die pharmazeutische Anwendung in ihre physiologisch verträglichen Salze, überführt werden. Als Basen kommen hierbei beispielsweise Natriumhydroxid, Kaliumhydroxid, Calciumhydroxid, Lysin, Cyclohexylamin, Ethanolamin, Diethanolamin und Triethanolamin in Betracht.

Gemäß einer bevorzugten Ausführungsform werden Indol-Derivate gemäß der vorstehenden allgemeinen Formel (1) bereitgestellt, welche dadurch gekennzeichnet sind, dass R, R2, R3, R4, Z1 und Z2 die vorstehend genannten Bedeutungen besitzen und
- R₁: einen 2-, 4-, 5- oder 6-Pyrimidinyl-Rest oder 2-, 4-, 5- oder 6-Pyrimidinyl-(C₁-C₄)-alkyl-Rest, wobei der (C₁ -C₄)-alkyl-Rest unsubstituiert oder einmehrfach gleich oder verschieden mit (C₁-C₆)-Alkyl, Halogen oder Oxo (=O) substituiert sein kann und der 2-, 4-, 5- oder 6-Pyrimidinyl-Rest jeweils unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann, *mit der Maßgabe,* dass R1 nicht für unsubstituiertes 2- oder 4-Pyrimidinyl oder einen ein- oder mehrfach mit der Methylgruppe substituierten 2-Pyrimidinyl-Rest steht;
einen 3-, 4-, 5- oder 6-Pyridazinyl-Rest oder 3-, 4-, 5-, oder 6-Pyridazinyl-(C₁ -C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 3-, 4-, 5-, oder 6-Pyridazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5- oder 6-Pyrazinyl-Rest oder 2-, 3-, 5,- oder 6-Pyrazinyl-(C₁-C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der 2-, 3-, 5,- oder 6-Pyrazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 3-, 4-, 5-, 6-, 7- oder 8-Cinnolinyl-Rest oder 3-, 4-, 5-, 6-, 7-, oder 8-Cinnolinyl-(C₁ -C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der 3-, 4-, 5-, 6-, 7-, oder 8-Cinnolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6-, 7- oder 8-Chinazolinyl-Rest oder 2-, 4-, 5-, 6-, 7-, oder 8-Chinazolinyl-(C₁ -C₄)-alkyl-Rest, wobei der (C₁ -C₄)-alkyl-Rest unsubstituiert oder ein- oder mehrfach gleich oder verschieden mit Wasserstoff, (C₁-C₆)-Alkyl, Halogen oder Oxo (=O) substituiert sein kann und der oder 2-, 4-, 5-, 6-, 7-, oder 8-Chinazolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5-, 6-, 7- oder 8-Chinoxalinyl-Rest oder 2-, 3-, 5-, 6-, 7- oder 8-Chinoxalinyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der oder 2-, 3-, 5-, 6-, 7- oder 8-Chinoxalinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 4-, 5-, 6-, 7- oder 8-Phthalazinyl-Rest oder 1-, 4-, 5-, 6-, 7- oder 8-Phthalazinyl-(C₁ -C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der oder 1-, 4-, 5-, 6-, 7- oder 8-Phthalazinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, 4-, 5-, 6-, 7- oder 8-Isochinolyl- oder 1-, 3-, 4-, 5-, 6-, 7- oder 8-Isochinolyl-(C₁ -C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4-, 5-, 6-, 7- oder 8-Isochinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 6-, 8- oder 9-[9*H*]-Purinyl-Rest oder 2-, 6-, 8- oder 9-[9*H*]-Purinyl-(C₁-C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 2-, 6-, 8- oder 9-[9*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 6-, 7- oder 8-[7*H*]-Purinyl-Rest oder 2-, 6-, 7- oder 8-[7*H*]-Purinyl-(C₁ -C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 2-, 6-, 7- oder 8-[7*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 3-, 4-, 5-, 6-, 7-, 8- oder 9-Acridinyl- oder 1-, 2-, 3-, 4-, 5-, 6-, 7-, 8- oder 9-Acridinyl-(C₁-C₄)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6-, 7-, 8- oder 9-Acridinyl-Rest unsubstituiert oder ein- bis achtfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 3-, 4-, 6-, 7-, 8-, 9- oder 10-Phenanthridinyl- oder 1-, 2-, 3-, 4-, 6-, 7-, 8- oder 9- oder 10-Phenanthridinyl-(C₁ -C₆)-alkyl-Rest, wobei der (C₁ -C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 2-, 3-, 4-, 6-, 7-, 8-, 9- oder 10-Phenanthridinyl-Rest unsubstituiert oder ein- bis achtfach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, (C₆-C₁₀)-Aryl-(C₁-C₆)-alkoxy, vorzugsweise Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 3-, 4-, 5,- oder 6-Pyridyl-Rest, wobei der 2-, 3-, 4-, 5,- oder 6-Pyridyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann, *mit der Maßgabe,* dass Z₁, Z₂, R₂, R₃ und R₄ ohne Einschränkung die vorstehend oder nachfolgend beschriebene Bedeutung haben und R für (C₂-C₆)-Alkenyl, vorzugsweise Allyl, (C₂-C₆)-Alkinyl, vorzugsweise Ethinyl oder Propargyl, (C₁-C₆)-Alkoxy-(C₁-C₆)-alkyl, oder geradkettiges oder verzweigtes Cyano-(C₁-C₆)-Alkyl, vorzugsweise Cyanomethyl, steht *oder mit der Maßgabe,* dass Z₂, R, R₂, R₃ und R₄ ohne Einschränkung die vorstehend oder nachfolgend beschriebenen Bedeutungen haben und Z₁ geminal verknüpftes Wasserstoff und Hydroxy bedeutet;
einen 2-, 3-, 4-, 5,- oder 6-Pyridyl-(C₁ -C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5,- oder 6-Pyridyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 3-, 4,- oder 5-Thienyl-Rest oder 2-, 3-, 4,- oder 5-Thienyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4,- oder 5-Thienyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 4-, oder 5-Thiazolyl-Rest oder 2-, 4-, oder 5-Thiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Thiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, oder 5-Isothiazolyl-Rest oder 3-, 4- oder 5-Isothiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4- oder 5-Isothiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6- oder 7-Benzothiazolyl-Rest oder 2-, 4-, 5-, 6- oder 7-Benzo-thiazolyl (C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, 5-, 6-oder 7-Benzothiazolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 4- oder 5-Imidazolyl-Rest oder 1-, 2-, 4- oder 5-Imidazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 4- oder 5-Imidazolyl -Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, 4- oder 5-Pyrazolyl-Rest oder 1-, 3-, 4- oder 5-Pyrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4- oder 5-Pyrazolyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2,- 3,- 4,- oder 5-Pyrrolyl-Rest oder 1-, 2,- 3,- 4,- oder 5-Pyrrolyl-(C₁-C₆)-alkyl-Rest, wobei der (C₁-C₆)-alkyl-Rest unsubstituiert oder ein-oder mehrfach gleich oder verschieden mit (C₁-C₆)-Alkyl, Halogen oder Oxo (=O) substituiert sein kann und der 1-, 2,- 3,- 4,- oder 5-Pyrrolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, oder 5-[1.2.4]-Triazolyl-Rest oder 1-, 3-, oder 5-[1.2.4]-Triazolyl-(C₁-C₆)-alkyl-Rest, wobei der (C₁ -C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 3- oder 5-[1.2.4]-Triazolyl-Rest unsubstituiert oder ein- oder zweifach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 4- oder 5-[1.2.3]-Triazolyl-Rest oder 1-, 4- oder 5-[1.2.3]-Triazolyl-(C₁-C₆)-alkyl-Rest, wobei der (C₁-C₆)-alkyl-Rest unsubstituiert oder ein-oder mehrfach gleich oder verschieden mit (C₁-C₆)-Alkyl, Halogen oder Oxo (=O) substituiert sein kann und der 1-, 4- oder 5-[1.2.3]-Triazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1- oder 5-[1 *H*]-Tetrazolyl-Rest oder 1- oder 5-[1 *H*]-Tetrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1- oder 5-[1 *H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2- oder 5-[2*H*]-Tetrazolyl-Rest oder 2- oder 5-[2*H*]-Tetrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2- oder 5-[2*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4- oder 6-[1.3.5]-Triazinyl-Rest oder 2-, 4- oder 6-[1.3.5]-Triazinyl-(C₁-C₆)-alkyl-Rest, wobei der (C₁-C₆)-alkyl-Rest unsubstituiert oder ein-oder mehrfach gleich oder verschieden mit Wasserstoff, (C₁-C₆)-Alkyl, Halogen oder Oxo (=O) substituiert sein kann und der 2-, 4- oder 6-[1.3.5]-Triazinyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4- oder 5-Oxazolyl-Rest oder 2-, 4- oder 5-Oxazolyl-(C₁-C₆)-alkylRest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Oxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 3-, 4- oder 5-Isoxazolyl-Rest oder 3-, 4- oder 5-Isoxazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4- oder 5-Isoxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 3-, 4-, 5-, 6- oder 7-Indolyl-Rest oder 1-, 2-, 3-, 4-, 5-, 6- oder 7-Indolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6- oder 7-Indolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann; bedeutet,
sowie die Isomeren, insbesondere Tautomere, Diastereomere und Enantiomere, und den pharmazeutisch verträglichen Salzen, insbesondere Säureadditionssalze, davon.

Gemäß einem weiteren Aspekt der Erfindung wird ein Verfahren zur Herstellung von Indolderivaten gemäß der allgemeinen Formel (1) nach einem der Ansprüche 1 und 2 dadurch gekennzeichnet, dass eine Indolvorstufe der allgemeinen Formel (2), 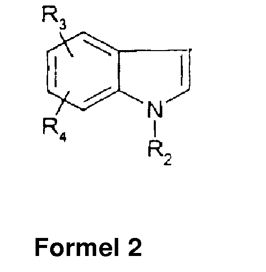 worin R2, R3 und R4 die vorstehend genannten Bedeutungen besitzen, mit einer Verbindung der allgemeinen Formel (3) 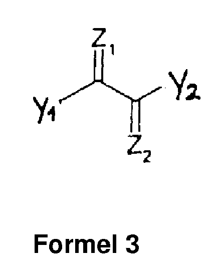 worin Z1 und Z2 die vorstehend genannte Bedeutung besitzen und Y1 und Y2 unabhängig voneinander für eine geeignete Abgangsgruppe wie Halogen, (C1-C6)-Alkoxy, -O-Tosyl, -O-Mesyl oder- N1-imidazol steht, und anschließend mit einem Amin der allgemeinen Formel (4) oder (5), 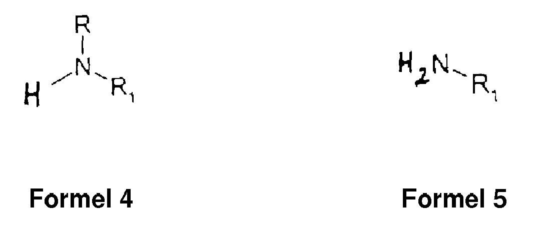 worin R und R1 die vorstehend genannten Bedeutungen haben, unter Bildung des gewünschten Indolderivates der allgemeinen Formel (1) (mit Amin 4) oder der Verbindung der allgemeinen Formel (6) (mit Amin 5) 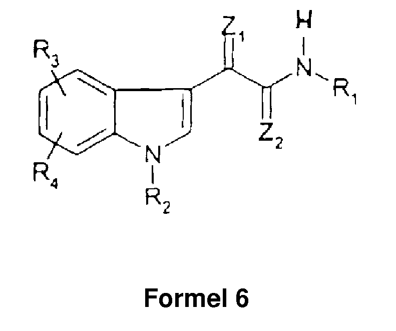 worin R1, R2, R3, R4, Z1 und Z2 die vorstehend genannten Bedeutungen besitzen, gegebenenfalls unter Verwendung von Verdünnungs- und Hilfsmitteln umgesetzt wird, wobei anschließend die Verbindung der allgemeinen Formel (6) mit einer Verbindung der allgemeinen Formel (7)

R-Y3 (7)

worin R die vorstehend genannte Bedeutung besitzt und Y3 für eine geeignete elektrophile Abgangsgruppe wie Halogen, (C1-C6)-Alkoxy, -O-Tosyl, -O-Mesyl oder -N1-imidazol steht, unter Erhalt der gewünschten Verbindung (1), wobei R nicht Wasserstoff bedeutet, gegebenenfalls unter Verwendung von Verdünnungs-und Hilfsmitteln umgesetzt werden kann.

### Synthesewege:

Die Verbindungen der allgemeinen Formel 1 sind dem folgenden Schema 1 gemäß erhältlich:

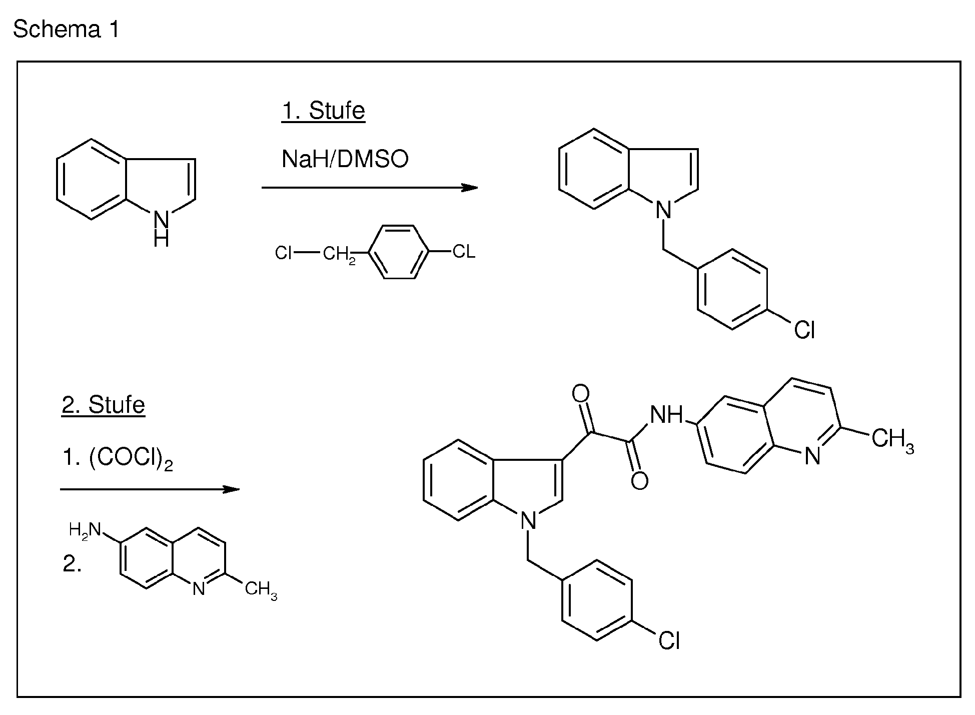

Weiterhin sind die Verbindungen der allgemeinen Formel 1 auch noch nach dem Syntheseweg des Schemas 2 erhältlich:

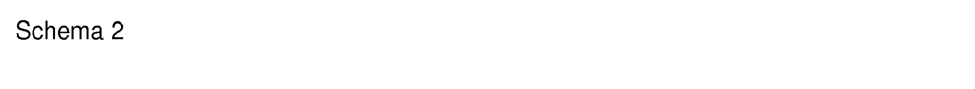

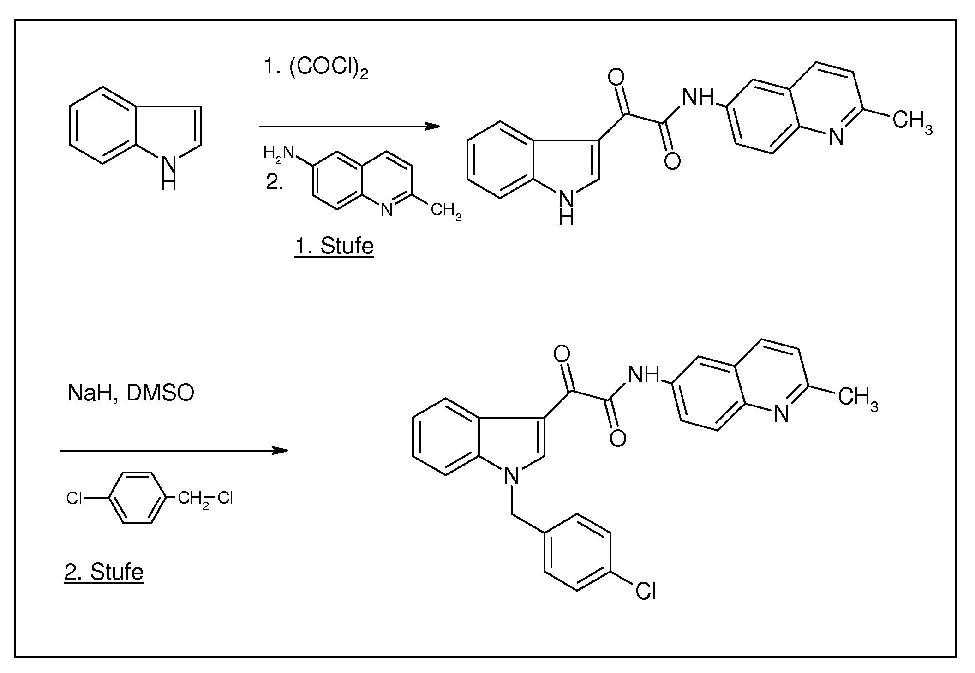

Die Verbindungen der allgemeinen Formel 1 bei denen R= die Methylgruppe, der Benzylrest, die Propargylgruppe oder der Cyanomethyl-Rest ist und R₁, R₂, R₃ und R₄ der allgemeinen Formel 1 die angegebenen Bedeutungen besitzen, können auch nach dem Syntheseweg des Schemas 3 hergestellt werden:

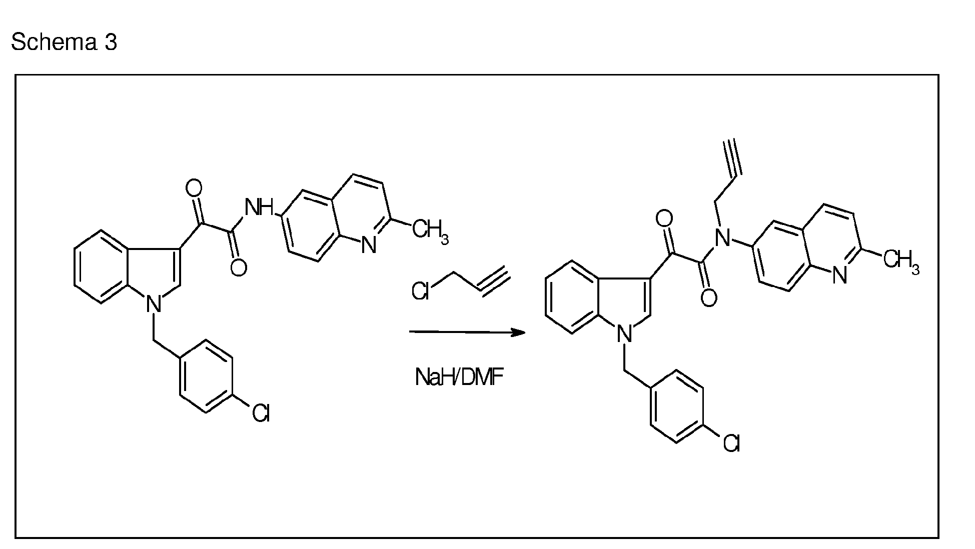

Die Ausgangsverbindungen (2), (3) und (4) sind entweder im Handel erhältlich oder können nach an sich bekannten Verfahrensweisen hergestellt werden. Die Edukte (2), (3) und (4) stellen wertvolle Zwischenverbindungen für die Herstellung der erfindungsgemäßen Indolderivate der Formel (1) dar.

Für die Herstellung der Ausgangs- und Zielverbindungen sei beispielsweise auf folgende Standardwerke der organischen Synthese verwiesen, dessen Inhalt hiermit Bestandteil der Offenbarung der vorliegenden Anmeldung werden soll:
1) Houben-Weyl, Band E 7a (Teil1) S. 290-492, S.571-740
   Houben-Weyl, Band E 7a (Teil 2) S. 119-156, S.205-686, S. 157-204
2) Monographie "Heterocyclic Compounds" (Elderfield), Volume 1, S.119- 207, S. 397-616
   Volume 3, S. 1-274
   Volume 6, S. 101-135, S. 234-323
3) Monographie "Comprehensive Organic Chemistry" (S.D.Barton, W.D. Ollis) Volume 4, S.155-204, S.205-232, S.493-564

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.
Die erfindungsgemäßen Indolderivate gemäß der allgemeinen Formel (1) sind als Arzneimittel, insbesondere als Antitumormittel, zur Behandlung von Säugetieren, insbesondere dem Menschen, aber auch für Haustiere wie Pferde, Kühe, Hunde, Katzen, Hasen, Schafe, Geflügel und dergleichen geeignet.

Gemäß einem weiteren Aspekt der Erfindung wird ein Verfahren zur Bekämpfung von Tumoren in Säugetieren, insbesondere beim Menschen bereit gestellt, welches dadurch gekennzeichnet ist, dass mindestens ein Indol-Derivat gemäß der allgemeinen Formel (1) einem Säugetier in einer für die Tumorbehandlung wirksamen Menge verabreicht wird. Die für die Behandlung zu verabreichende therapeutisch effektive Dosis des jeweiligen erfindungsgemäßen Indol-Derivates richtet sich u. a. nach der Art und dem Stadium der Tumorerkrankung, dem Alter und Geschlecht des Patienten, der Art der Verabreichung und der Dauer der Behandlung. Die Verabreichung kann oral, rectal, buccal (z.B. sublingual), parenteral (z. B. subkutan, intramuskulär, intradermal oder intravenös), topisch oder transdermal erfolgen.

Gemäß einem weiteren Aspekt der Erfindung werden Arzneimittel zur Tumorbehandlung bereitgestellt, welche dadurch gekennzeichnet sind, dass sie als wirksamen Bestandteil mindestens ein Indol-Derivat nach einem der Ansprüche 1 und 2 oder einem pharmazeutisch verträglichen Salz davon, gegebenenfalls zusammen mit üblichen pharmazeutisch verträglichen Hilfs-, Zusatz- und Trägerstoffen enthalten. Es kann sich dabei um feste, halbfeste, flüssige oder Aerosol-Zubereitungen handeln. Geeignete feste Zubereitungen sind beispielsweise Kapseln, Pulver, Granulate, Tabletten. Geeignete halbfeste Zubereitungen sind beispielsweise Salben, Cremes, Gele, Pasten, Suspensionen, Öl-in-Wasser- und Wasser-in-Öl-Emulsionen. Geeignete flüssige Zubereitungen sind beispielsweise sterile wässrige Zubereitungen für die parenterale Verabreichung, die isoton mit dem Blut des Patienten sind.

Die Erfindung soll anhand des nachfolgenden Beispiels näher erläutert werden, ohne darauf beschränkt zu sein.

### Ausführungsbeispiele

### Beispiel 1 (Umsetzung gemäß Schema 1, 1, Stufe):

### Herstellung von 1-(4-Chlorbenzyl)-indol

In eine Mischung von 1,32 g Natriumhydrid (0,055 Mol, Mineralölsuspension) in 50 ml Dimethylsulfoxid wird eine Lösung von 5,86 g (0,05 Mol) Indol in 25 ml DMSO gegeben. Man erhitzt 1,3 Stunden auf 60 °C, lässt danach abkühlen und tropft 17,7 g (0,11 Mol) 4-Chlor-benzylchlorid zu. Man erwärmt die Lösung auf 60 °C, lässt über Nacht stehen und gießt sodann unter Rühren in 200 ml Wasser. Man extrahiert mehrmals mit insgesamt 75 ml CH₂Cl₂, trocknet die organische Phase mit wasserfreiem Natriumsulfat, filtriert und engt das Filtrat im Vakuum ein.
Ausbeute: 11,5g (95 % d. Th.)

### Beispiel 2 (Umsetzung gemäß 2. Stufe von Schema 1):

### Herstellung von N-(2-Methyl-6-chinolyl)-[1-(4-chlorbenzyl)-indol-3-yl]-glyoxylsäureamid (D-69429)

Zu einer Lösung von 5,50 ml Oxalylchlorid in 50 ml Ether wird bei 0°C und unter Stickstoff tropfenweise eine Lösung von 10,2 g (42,2 mMol) 1-(4-Chlorbenzyl)-indol in 50 ml Ether gegeben. Man erhitzt 2 Stdn. zum Rückfluss und dampft anschließend das Lösungsmittel ab. Sodann wurden zum Rückstand 100 ml Tetrahydrofuran zugefügt, die Lösung auf -4°C gekühlt und tropfenweise mit einer Lösung von 15,66 g (99,0 mMol) 6-Amino-2-methyl-chinolin in 350 ml THF versetzt. Man erhitzt 4 Stunden am Rückfluss und lässt über Nacht bei Raumtemperatur stehen. Das 6-Amino-2-methyl-chinolin Hydrochlorid wird abgesaugt, der Niederschlag mit THF gewaschen, das Filtrat im Vakuum eingeengt und der Rückstand aus Methylethylketon/Methylenchlorid umkristallisiert.
Ausbeute: 14,8 g (77,3 % d. Th.)
Schmelzpunkt: 182-185 °C

### Herstellung des Hydrochlorids:

Zu 0,453 g (1 mmol) N-(2-Methyl-6-chinolyl)-[1-4-chlorbenzyl)-indol-3-yl]-glyoxylsäureamid in 20 ml heißem Ethanol wird die äquivalente Menge isopropanolische Salzsäure gegeben, das Gemisch auf 70-80 °C erhitzt und die Lösung i. Vak. bei 40 °C zur Trockne eingeengt. Mehrmaliges Nachdampfen mit Toluol ergab das farblose und kristalline Hydrochlorid.
Ausbeute: 0,49 g (100 % d. Th.)
Fp.: 196 °C

### Herstellung des Methansulfonats:

Ein Gemisch von 0,453 g (1 mMol) N-(2-Methyl-6-chinolyl)-[1-(4-chlorbenzyl)-indol-3-yl]-glyoxylsäureamid und 0,67 ml Methansulfonsäure in 15 ml Dichlormethan wird 30 Minuten auf 50 °C erwärmt, die gebildete Lösung i. Vak. bei 35 °C eingeengt, der Rückstand mehrmals mit Methyl-tert.-butylether nachgedampft und der verbleibende Rückstand bei 35 °C i. Vak. getrocknet.
Ausbeute: 0,46 g (84 % d. Th.)
Fp.: > 230 °C

### Beispiel 3 (Umsetzung gemäß Schema 3):

### Herstellung von N-Propargyl-N-(2-methyl-6-chinolyl)-[1-(4-chlorbenzyl)-indol-3-yl]-glyoxylsäureamid

Zu einer Suspension von 0,154 g Natriumhydrid (5,13 mMol, Mineralölsuspension) in 10 ml DMF wird bei Raumtemperatur und unter Stickstoff eine Suspension von 2,32 g (5,13 mMol) N-(2-Methyl-6-chinolyl)-[1-(4-chlorbenzyl)-indol-3-yl]-glyoxylsäureamid in 20 ml DMF gegeben. Es erfolgte unter Gelbfärbung ein starkes Aufschäumen. Man tropft eine Lösung von 0,382 g (5,13 mmol) Propargylchlorid in 10 ml DMF zu, rührt 24 Stdn. bei Raumtemperatur unter Stickstoff-Begasung und lässt 4 Tage bei Raumtemperatur stehen. Die dunkelbraune Lösung wird sodann auf 120 ml Eiswasser gegossen, die Lösung portionsweise mit 250 ml Methylenchlorid extrahiert und die vereinigten organischen Phasen mit wasserfreiem Natriumsulfat getrocknet. Man engt i. Vak. ein, reinigt den Rückstand über eine Kieselgelsäule (Kieselgel 60, Fa. Merck AG, Darmstadt) unter Anwendung des Elutionsmittels Methylenchlorid/Ethanol (97:3, V/V).
Ausbeute: 1,98 g (78,6 % d. Th.)
Mass spectrum: m/e= 491,9 (M⁺)

### Biologische Untersuchungen

### 1. Anti-proliferative Wirkung an verschiedenen Tumorzelllinien

Die Substanz D-69429 wurde in einem Proliferationstest an etablierten Tumorzelllinien auf ihre anti-proliferative Aktivität hin untersucht. Der verwendete Test bestimmt die zelluläre Dehydrogenaseaktivität und ermöglicht eine Bestimmung der Zellvitalität und indirekt der Zellzahl. Bei den verwendeten Zelllinien handelt es sich um die humane Cervixkarzinomzelllinie KB / HeLa (ATCC CCL17), die murine lymphozytäre Leukämie L1210 (ATCC CCL-219), die humane Brustadenokarzinomlinie MCF7 (ATCC HTB22) und die ovariale Adenokarzinomlinie SKOV-3 (ATCC HTB77). Es handelt sich hierbei um sehr gut charakterisierte, etablierte Zelllinien, die von ATCC erhalten und in Kultur genommen wurden.

Die in Tab. 1 gezeigten Ergebnisse belegen eine sehr potente anti-proliferative Wirkung von D-69429 an den Zelllinien SKOV-3, L-1210 und HeLa/KB. Aufgrund der Besonderheit des langsamen Wachstums der MCF7 Linie ist die Wirkung von D-69429 im Versuchszeitraum von 48 h nur gering (18 % Hemmung bei 3.16 µg/ml; daher Angabe >3.16).

### 2. Methode

### XTT-Test auf zelluläre Dehydrogenaseaktivität

Die adherent wachsenden Tumorzelllinien HeLa/KB, SKOV-3 und MCF7 sowie die in Suspension wachsende L1210 Leukämielinie wurden unter Standardbedingungen im Begasungsbrutschrank bei 37 °C, 5 % CO₂ und 95 % Luftfeuchtigkeit kultiviert. Am Versuchstag 1 werden die adherenten Zellen mit Trypsin / EDTA abgelöst und durch Zentrifugation pelletiert. Nachfolgend wird das Zellpellet im RPMI Kulturmedium in der entsprechenden Zellzahl resuspendiert und in eine 96-well Mikrotiterplatte umgesetzt. Die Platten werden dann über Nacht im Begasungsbrutschrank kultiviert. Die Testsubstanzen werden als Stammlösungen in DMSO angesetzt und am Versuchstag 2 mit Kulturmedium in den entsprechenden Konzentrationen verdünnt. Die Substanzen in Kulturmedium werden dann zu den Zellen gegeben und für 45h im Begasungsbrutschrank inkubiert. Als Kontrolle dienen Zellen, die nicht mit Testsubstanz behandelt werden.

Für das XTT-Assay werden 1mg/ml XTT (Natrium 3'-[1-(phenylaminocarbonyl)-3,4-tetrazolium]-bis(4-methoxy-6-nitro)benzensulfonsäure) in RPMI-1640 Medium ohne Phenolrot gelöst. Zusätzlich wird eine 0,383 mg/ml PMS (N-Methyl Dibenzopyrazine Methylsulfat) Lösung in Phosphat-gepufferter Salzlösung (PBS) hergestellt. Am Versuchstag 4 wird auf die Zellplatten, die inzwischen 45 h mit den Testsubstanzen inkubiert wurden, 75 µl/well XTT-PMS-Mischung pipettiert. Dazu wird kurz vor Gebrauch die XTT-Lösung mit der PMS-Lösung im Verhältnis 50:1 (Vol:Vol) gemischt. Anschließend werden die Zellplatten im Begasungsbrutschrank für weitere 3 h inkubiert und im Photometer die optische Dichte (OD₄₉₀ₙₘ) bestimmt.
Mittels der bestimmten OD₄₉₀ₙₘ wird die prozentuale Hemmung relativ zur Kontrolle berechnet. Die anti-proliferative Wirkung wird mittels einer Regressionsanalyse abgeschätzt.

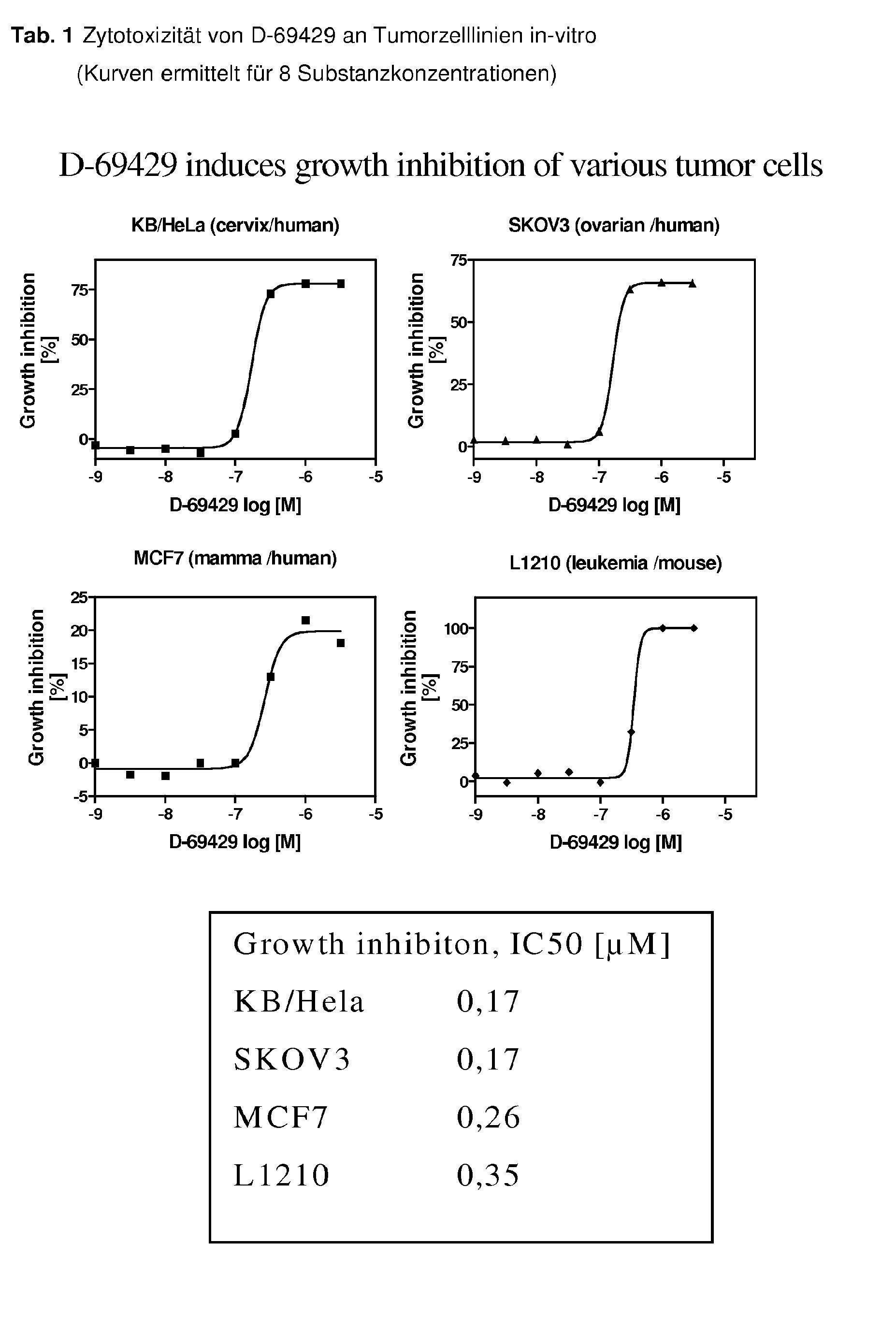

### Beispiel I

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| Summe: | 215,0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst.

### Beispiel II

Kapsel mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| Summe: | 160,0 mg |

### Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

## Patentansprüche

1. Indol-Derivate der allgemeinen Formel 1 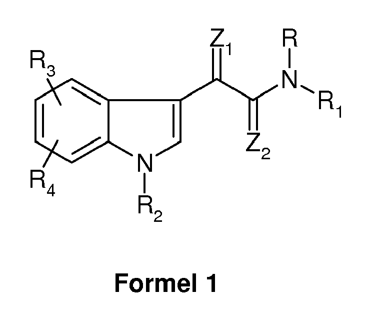 worin
R Wasserstoff; geradkettig oder verzweigtes (C₁-C₆)-Alkyl; ein- oder mehrfach durch Aryl substituiertes (C₁-C₆)-Alkyl, wobei der Arylrest unsubstituiert oder ein- bis fünffach gleich oder verschieden mit Halogen, (C₁-C₆)-Alkyl, (C₃-C₇)-Cycloalkyl, Carboxyl, (C₁-C₆)-Alkoxycarbonyl, vorzugsweise tertiärButoxycarbonyl, mit einem oder mehreren Fluoratomen substituiertes geradkettig oder verzweigtes (C₁-C₆)-Alkyl, vorzugsweise Trifluormethyl, Hydroxy, geradkettig oder verzweigtes (C₁-C₆)-Alkoxy, vorzugsweise Methoxy oder Ethoxy; Benzyloxy, oder Aryl-(C₁-C₆)-Alkyl, wobei der Arylrest unsubstituiert oder ein- oder fünffach gleich oder verschieden mit (C₁-C₆)-Alkyl, Halogen oder mit einem oder mehreren Fluoratomen substituiertes geradkettig oder verzweigtes (C₁-C₆)-Alkyl, vorzugsweise Trifluormethyl, substituiert sein kann; geradkettig oder verzweigtes (C₁-C₆)-Alkoy-(C₁-C₆)-Alkyl, substituiertes oder unsubstituiertes Aryl-(C₁-C₆)-alkyloxycarbonyl, vorzugsweise Benzyloxycarbonyl, geradkettig oder verzweigtes (C₁-C₆)-Alkyloxycarbonyl, geradkettig oder verzweigtes (C₁-C₆)-Alkylcarbonyl, vorzugsweise Acetyl, (C₂-C₆)-Alkenyl, vorzugsweise Allyl, (C₂-C₆)-Alkinyl, vorzugsweise Ethinyl oder Propargyl, oder geradkettiges oder verzweigtes Cyano-(C₁-C₆)-Alkyl, vorzugsweise Cyanomethyl, bedeutet;
R₁ einen ein oder mehrere Heteroatome ausgewählt aus der Gruppe N, O und S enthaltenden gesättigten, ungesättigten oder aromatischen (C2-C14)-Heterocyclus, welcher mit dem Amidstickstoff direkt oder über eine (C1-C6)-Alkylbrücke verbunden sein kann, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 4-, 5- oder 6-Pyrimidinyl-Rest jeweils unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann, ***mit der MaBgabe,*** dass R1 nicht für unsubstituiertes 2- oder 4-Pyrimidinyl oder einen ein- oder mehrfach mit der Methylgruppe substituierten 2-Pyrimidinyl-Rest steht; und ***ausgenommen*** einen 2-, 3-, 4-, 5-, 6-, 7- oder 8-Chinolyl-Rest oder 2-, 3-, 4-, 5-, 6-, 7- oder 8-Chinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, N,N-di-(C₁-C₆)-Alkylamino, Hydroxyl, (C₁-C₆)-Alkoxy, (C6-C14)-Aryl-(C1-C6)-alkyoxy, Carboxy, (C₁-C₆)-Alkoxycarbonyl, (C₁-C₆)-Alkoxycarbonylamino oder ein- oder mehrfach mit Halogen substituiertem (C₁ -C₆)-Alkyl, (C₆-C₁₄)-Aryl, oder (C₆-C₁₄)-Aryl-(C₁-C₆)-alkyl substituiert sein kann; bedeutet;
R₂ Wasserstoff, (C₁-C₆)-Alkyl, ein- oder mehrfach mit Halogen substituiertes (C₁-C₆)-Alkyl, ein- oder mehrfach mit Phenyl substituiertes (C₁-C₆)-Alkyl, wobei der Phenylrest unsubstituiert oder ein- bis fünffach gleich oder verschieden mit Halogen, vorzugsweise Chlor, Brom oder Jod, (C₁-C₆)-Alkyl, (C₃-C₇)-Cycloalkyl, Carboxyl, (C₁-C₆)-Alkoxycarbonyl, ein- oder mehrfach mit Halogen substituiertem (C₁-C₆)-Alkyl, vorzugsweise Trifluormethyl, Hydroxy, (C₁-C₆)-Alkoxy, vorzugsweise Methoxy oder Ethoxy, Phenyl-(C₁-C₆)-Alkoxy, vorzugsweise Benzyloxy, Nitro, Amino, Mono-(C₁-C₆)-alkylamino, Di-(C₁-C₆)-alkylamino, Mono-(C₃₋₆)-cycloalkylamino, Di-(C₃₋₆)-cycloalkylamino, (C₁-C₆)-Acylamino, Phenyl-(C₁-C₆)-alkylamino, Arylcarbonylamino, Heteroarylcarbonylamino, (C₁-C₆)-Alkylsulfonamido, Arylsulfonamido, Maleinimido, Succinimido, Phthalimido, Benzyloxycarbonylamino (Z-Amino), tert.-Butoxycarbonylamino (BOC-Amino), 9-Fluorenylmethoxy-carbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), mit -NH-CH₂-COOH; -NH-CH(CH₃)-COOH; (CH₃)₂CH-CH₂-CH₂-CH(NH-)-COOH; H₃C-CH₂-CH(CH₃)-CH(NH-)-COOH;
HOH₂C-CH(NH-)-COOH; Phenyl-CH₂-CH(NH-)-COOH; (4-Imidazolyl)-CH₂-CH(NH-)-COOH; HN=C(NH₂)-NH-(CH₂)₃-CH(NH-)-COOH; H₂N-(CH₂)₄-CH(NH-)-COOH;
H₂N-CO-CH₂-CH(NH-)-COOH; HOOC-(CH₂)₂-CH(NH-)-COOH substituiert sein kann;
einen 2-, 3-, 4-, 5-, 6-, 7- und 8-Chinolyl-(C₁-C₆)-Alkyl-Rest, wobei der 2-, 3-, 4-, 5,-, 6- , 7- und 8-Chinolyl-Rest unsubstituiert oder ein- bis sechsfach gleich oder verschieden mit Halogen, (C₁-C₄)-Alkyl oder (C₁-C₄)-Alkoxy substituiert sein kann;
einen 2-, 3- und 4-Pyridyl-(C₁-C₆)-Alkyl-Rest, wobei der 2-, 3- und 4-Pyridyl-Rest unsubstituiert oder ein- bis vierfach gleich oder verschieden mit Halogen, (C₁-C₄)-Alkyl oder (C₁-C₄)-Alkoxy substituiert sein kann;
einen Arylcarbonyl-Rest, wobei der Aryl-Rest unsubstituiert oder ein- bis fünffach gleich oder verschieden mit Halogen, (C₁-C₆)-Alkyl, (C₃-C₇)-Cycloalkyl, Carboxyl, Cyano, (C₁-C₆)-Alkoxycarbonyl, ein- oder mehrfach mit Fluoratomen substituiertes (C₁-C₆)-Alkyl vorzugsweise Trifluormethyl, Hydroxyl, (C₁-C₄)-Alkoxy vorzugsweise Methoxy oder Ethoxy, Aryl-(C₁-C₄)-Alkoxy, vorzugsweise Benzyloxy, substituiert sein kann; bedeutet;
R₃ und R₄ können an die Indolkohlenstoffatome C-2, C-4, C-5, C-6 oder C-7 gebunden sein und unabhängig voneinander Wasserstoff, Hydroxy, (C₁-C₆)-Alkyl, (C₃-C₇)-Cycloalkyl, (C₁-C₆)-Alkylcarbonyl, (C₁-C₆)-Alkoxy, Halogen, Aryl-(C₁-C₄)-Alkoxy, vorzugsweise Benzyloxy, Nitro, Amino, Mono-(C₁-C₄)-Alkyl-amino, Di-(C₁-C₄)-Alkylamino, (C₁-C₆)-Alkoxycarbonylamino, (C₁-C₆)-Alkoxycarbonylamino-(C₁-C₆)-alkyl, Cyano, geradkettiges oder verzweigtes Cyano-(C₁-C₆)-alkyl, Carboxyl, (C₁-C₄)-Alkoxycarbonyl, mit ein oder mehreren Fluoratomen substituiertes (C₁-C₄)-Alkyl, vorzugsweise Trifluormethylgruppe, Carboxy-(C₁-C₆)-alkyl oder (C₁-C₆)-Alkoxycarbonyl-(C₁-C₆)-alkyl bedeuten;
Z₁ Sauerstoff oder Schwefel oder geminal verknüpftes Wasserstoff und Hydroxy bedeutet;
Z₂ Sauerstoff oder Schwefel bedeutet,
deren Tautomere, Stereoisomere, einschließlich der Diastereomere und Enantiomere, sowie die physiologisch verträglichen Salze davon.

2. Indol-Derivate gemäß der allgemeinen Formel (1) nach Anspruch 1, **dadurch gekennzeichnet, dass** R, R2, R3, R4, Z1 und Z2 die vorstehend genannten Bedeutungen besitzen und
R₁ einen 2-, 4-, 5- oder 6-**Pyrimidinyl**-Rest oder 2-, 4-, 5- oder 6-Pyrimidinyl-(C₁ -C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 2-, 4-, 5- oder 6-Pyrimidinyl-Rest jeweils unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann, ***mit der Maßgabe,*** dass R1 nicht für unsubstituiertes 2- oder 4-Pyrimidinyl oder einen ein- oder mehrfach mit der Methylgruppe substituierten 2-Pyrimidinyl-Rest steht;
einen 3-, 4-, 5- oder 6-**Pyridazinyl**-Rest oder 3-, 4-, 5-, oder 6-Pyridazinyl-(C₁ -C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 3-, 4-, 5-, oder 6-Pyridazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5- oder 6-Pyrazinyl-Rest oder 2-, 3-, 5,- oder 6-Pyrazinyl-(C₁-C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der 2-, 3-, 5,- oder 6-Pyrazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 3-, 4-, 5-, 6-, 7- oder 8-**Cinnolinyl**-Rest oder 3-, 4-, 5-, 6-, 7-, oder 8-Cinnolinyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 3-, 4-, 5-, 6-, 7-, oder 8-Cinnolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6-, 7- oder 8-**Chinazolinyl**-Rest oder 2-, 4-, 5-, 6-, 7-, oder 8-Chinazolinyl-(C₁ -C₄)-alkyl-Rest, wobei der (C₁ -C₄)-alkyl-Rest unsubstituiert oder ein- oder mehrfach gleich oder verschieden mit Wasserstoff, (C₁-C₆)-Alkyl, Halogen oder Oxo (=O) substituiert sein kann und der oder 2-, 4-, 5-, 6-, 7-, oder 8-Chinazolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5-, 6-, 7- oder 8-**Chinoxalinyl-**Rest oder 2-, 3-, 5-, 6-, 7- oder 8-Chinoxalinyl-(C₁ -C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der oder 2-, 3-, 5-, 6-, 7- oder 8-Chinoxalinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 4-, 5-, 6-, 7- oder 8-**Phthalazinyl**-Rest oder 1-, 4-, 5-, 6-, 7- oder 8-Phthalazinyl-(C₁ -C₄)-alkyl-Rest, wobei der (C₁ -C₄)-alkyl-Rest unsubstituiert oder ein- oder mehrfach gleich oder verschieden mit (C₁-C₆)-Alkyl, Halogen oder Oxo (=O) substituiert sein kann und der oder 1-, 4-, 5-, 6-, 7- oder 8-Phthalazinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, 4-, 5-, 6-, 7- oder 8-**Isochinolyl**- oder 1-, 3-, 4-, 5-, 6-, 7- oder 8-Isochinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4-, 5-, 6-, 7- oder 8-Isochinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 6-, 8- oder 9**-[9*H*]-Purinyl-**Rest oder 2-, 6-, 8- oder 9-[9*H*]-Purinyl-(C₁ -C₄)-alkyl-Rest, wobei der (C₁ -C₄)-alkyl-Rest unsubstituiert oder ein-oder mehrfach gleich oder verschieden mit (C₁-C₆)-Alkyl, Halogen oder Oxo (=O) substituiert sein kann und der 2-, 6-, 8- oder 9-[9*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 6-, 7- oder 8**-[7*H*]-Purinyl**-Rest oder 2-, 6-, 7- oder 8-[7*H*]-Purinyl-(C₁-C₄)-alkyl-Rest, wobei der (C₁-C₄)-alkyl-Rest unsubstituiert oder ein-oder mehrfach gleich oder verschieden mit (C₁-C₆)-Alkyl, Halogen oder Oxo (=O) substituiert sein kann und der 2-, 6-, 7- oder 8-[7*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 3-, 4-, 5-, 6-, 7-, 8- oder 9-**Acridinyl-** oder 1-, 2-, 3-, 4-, 5-, 6-, 7-, 8- oder 9-Acridinyl-(C₁ -C₄)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6-, 7-, 8- oder 9-Acridinyl-Rest unsubstituiert oder ein- bis achtfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 3-, 4-, 6-, 7-, 8-, 9- oder 10-**Phenanthridinyl**- oder 1-, 2-, 3-, 4-, 6-, 7-, 8- oder 9- oder 10-Phenanthridinyl-(C₁ -C₆)-alkyl-Rest, wobei der (C₁ -C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 2-, 3-, 4-, 6-, 7-, 8-, 9- oder 10-Phenanthridinyl-Rest unsubstituiert oder ein- bis achtfach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, (C₆-C₁₀)-Aryl-(C₁-C₆)-alkoxy, vorzugsweise Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 3-, 4-, 5,- oder 6-**Pyridyl**-Rest, wobei der 2-, 3-, 4-, 5,- oder 6-Pyridyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann, ***mit der Maßgabe,*** dass Z₁, Z₂, R₂, R₃ und R₄ ohne Einschränkung die vorstehend oder nachfolgend beschriebene Bedeutung haben und R für (C₂-C₆)-Alkenyl, vorzugsweise Allyl, (C₂ -C₆)-Alkinyl, vorzugsweise Ethinyl oder Propargyl, (C₁-C₆)-Alkoxy-(C₁-C₆)-alkyl, oder geradkettiges oder verzweigtes Cyano-(C₁-C₆)-Alkyl, vorzugsweise Cyanomethyl, steht ***oder mit der Maßgabe,*** dass Z₂, R, R₂, R₃ und R₄ ohne Einschränkung die vorstehend oder nachfolgend beschriebenen Bedeutungen haben und Z₁ geminal verknüpftes Wasserstoff und Hydroxy bedeutet;
einen 2-, 3-, 4-, 5,- oder 6-**Pyridyl-**(C₁-C₆)-**alkyl**-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5,- oder 6-Pyridyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 3-, 4,- oder 5-**Thienyl**-Rest oder 2-, 3-, 4,- oder 5-Thienyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4,- oder 5-Thienyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2-, 4-, oder 5-**Thiazolyl**-Rest oder 2-, 4-, oder 5-Thiazolyl-(C₁-C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Thiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, oder 5-**Isothiazolyl**-Rest oder 3-, 4- oder 5-Isothiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4- oder 5-Isothiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6- oder 7**-Benzothiazolyl**-Rest oder 2-, 4-, 5-, 6- oder 7-Benzothiazolyl (C₁ -C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, 5-, 6-oder 7-Benzothiazolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 2-, 4- oder 5-**Imidazolyl**-Rest oder 1-, 2-, 4- oder 5-Imidazolyl-(C₁ -C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 4- oder 5-Imidazolyl -Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, 4- oder 5-**Pyrazolyl**-Rest oder 1-, 3-, 4- oder 5-Pyrazolyl-(C₁-C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4- oder 5-Pyrazolyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2,- 3,- 4,- oder 5-**Pyrrolyl**-Rest oder 1-, 2,- 3,- 4,- oder 5-Pyrrolyl-(C₁-C₆)-alkyl-Rest, wobei der (C₁-C₆)-alkyl-Rest unsubstituiert oder ein-oder mehrfach gleich oder verschieden mit (C₁-C₆)-Alkyl, Halogen oder Oxo (=O) substituiert sein kann und der 1-, 2,- 3,- 4,- oder 5-Pyrrolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 3-, oder 5-**[1.2.4]-Triazolyl-**Rest oder 1-, 3-, oder 5-[1.2.4]-Triazolyl-(C₁ -C₆)-alkyl-Rest, wobei der (C₁ -C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 3- oder 5-[1.2.4]-Triazolyl-Rest unsubstituiert oder ein- oder zweifach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1-, 4- oder 5-[1.2.3]-**Triazolyl-**Rest oder 1-, 4- oder 5-[1.2.3]-Triazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 4- oder 5-[1.2.3]-Triazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 1- oder 5-[1 ***H*]-Tetrazolyl-**Rest oder 1- oder 5-[1 *H*]-Tetrazolyl-(C₁-C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 1- oder 5-[1 *H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 2- oder 5-**[2*H*]-Tetrazolyl**-Rest oder 2- oder 5-[2*H*]-Tetrazolyl-(C₁-C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 2- oder 5-[2*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4- oder 6-**[1.3.5]-Triazinyl**-Rest oder 2-, 4- oder 6-[1.3.5]-Triazinyl-(C₁ -C₆)-alkyl-Rest, wobei der (C₁ -C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 2-, 4- oder 6-[1.3.5]-Triazinyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4- oder 5-**Oxazolyl**-Rest oder 2-, 4- oder 5-Oxazolyl-(C₁-C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 2-, 4-, oder 5-Oxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl oder (C₆-C₁₀)-Aryl-(C₁-C₆)-alkyl substituiert sein kann;
einen 3-, 4- oder 5-**Isoxazolyl**-Rest oder 3-, 4- oder 5-Isoxazolyl-(C₁-C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 3-, 4- oder 5-Isoxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein-oder mehrfach mit Fluor substituiertem (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 3-, 4-, 5-, 6- oder 7-**Indolyl**-Rest oder 1-, 2-, 3-, 4-, 5-, 6- oder 7-Indolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6-oder 7-Indolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl oder (C₆-C₁₀)-Aryl-(C₁-C₆)-alkyl substituiert sein kann; bedeutet.

3. Indolderivate gemäß der allgemeinen Formel (1) nach einem der Ansprüche 1 oder 2 zur Verwendung als Arzneimittel.

4. Verwendung der Indolderivate gemäß der allgemeinen Formel (1) nach einem der Ansprüche 1 oder 2 zur Herstellung eines Arzneimittels zur Behandlung von Tumoren in Säugetieren.

5. Verfahren zur Herstellung von Indolderivaten gemäß der allgemeinen Formel (1) nach einem der Ansprüche 1 oder 2, **dadurch gekennzeichnet, dass** eine Indolvorstufe der allgemeinen Formel (2), 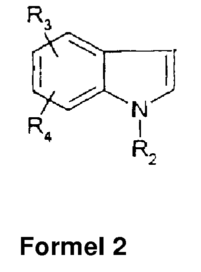 worin R2, R3 und R4 die vorstehend genannten Bedeutungen besitzen, mit einer Verbindung der allgemeinen Formel (3) 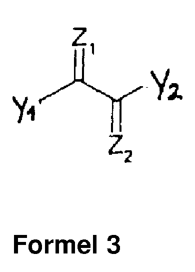 worin Z1 und Z2 die vorstehend genannte Bedeutung besitzen und Y1 und Y2 unabhängig voneinander für eine geeignete Abgangsgruppe wie Halogen, (C1-C6)-Alkoxy, -O-Tosyl, -O-Mesyl oder - N1-imidazol steht, und anschließend mit einem Amin der allgemeinen Formel (4) oder (5), 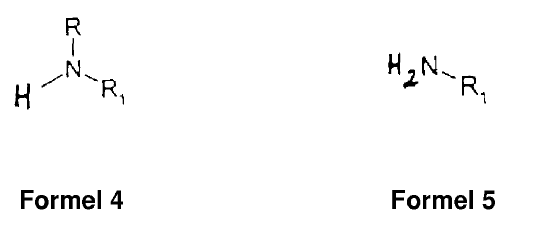 worin R und R1 die vorstehend genannten Bedeutungen haben, unter Erhalt der gewünschten Verbindung der allgemeinen Formel (1) (mit Amin 4) oder der Verbindung der allgemeinen Formel (6) (mit Amin 5) 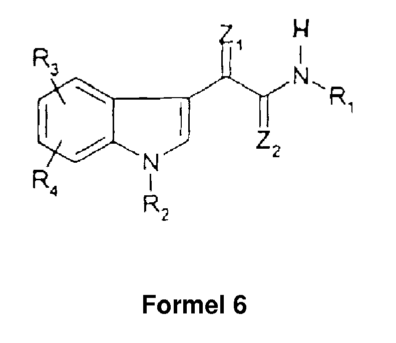 worin R1, R2, R3, R4, Z1 und Z2 die vorstehend genannten Bedeutungen besitzen, gegebenenfalls unter Verwendung von Verdünnungs- und Hilfsmitteln umgesetzt wird, wobei anschließend die Verbindung der allgemeinen Formel (6) mit einer Verbindung der allgemeinen Formel (7)
R-Y3 (7)
worin R die vorstehend genannte Bedeutung besitzt und Y3 für eine geeignete elektrophile Abgangsgruppe wie Halogen, (C₁-C₆)-Alkoxy, -O-Tosyl, -O-Mesyl oder -N1-imidazol steht, unter Erhalt der gewünschten Verbindung (1), wobei R nicht Wasserstoff bedeutet, gegebenenfalls unter Verwendung von Verdünnungs- und Hilfsmitteln umgesetzt werden kann.

6. Verfahren zur Behandlung von Tumoren in Säugetieren, **dadurch gekennzeichnet, dass** mindestens ein Indolderivat gemäß der allgemeinen Formel (1) nach einem der Ansprüche 1 oder 2 dem Säugetier in einer für die Tumorbehandlung wirksamen Dosis verabreicht wird.

7. Arzneimittel, **dadurch gekennzeichnet, dass** es als wirksamen Bestandteil mindestens ein Indolderivat nach einem der Ansprüche 1 oder 2 gegebenenfalls zusammen mit üblichen pharmazeutisch verträglichen Hilfs-, Zusatz- und Trägerstoffen enthält.
